Europäisches Patentamt

European Patent Office

Office européen des brevets

(19)

(11) Veröffentlichungsnummer : **0 468 195 A1**

## (12) EUROPÄISCHE PATENTANMELDUNG

(21) Anmeldenummer : **91109877.0**

(22) Anmeldetag : **17.06.91**

(51) Int. Cl.⁵ : **G21C 17/022, G21F 7/06, G01N 1/10**

(30) Priorität : **27.07.90 DE 4023841**

(43) Veröffentlichungstag der Anmeldung :
**29.01.92 Patentblatt 92/05**

(84) Benannte Vertragsstaaten :
**CH DE FR GB LI SE**

(71) Anmelder : **Deutsche Gesellschaft für Wiederaufarbeitung von Kernbrennstoffen mbH**
**Hamburger Allee 4 Postfach 1407**
**W-3000 Hannover 1 (DE)**

(72) Erfinder : **Zeh, Horst**
**Kolberger Str. 19b**
**W-7500 Karlsruhe (DE)**
Erfinder : **Dr.L. Finsterwalder**
**Strasse des Roten Kreuzes 11**
**W-7500 Karlsruhe 41 (DE)**

(74) Vertreter : **König, Norbert, Dipl.-Phys. Dr. et al**
**Patentanwälte Leine & König**
**Burckhardtstrasse 1**
**W-3000 Hannover 1 (DE)**

(54) **Einrichtung zur Entnahme von Gas- und/oder Flüssigkeitsproben aus einem Sicherheitsbehälter von Kernkraftwerken.**

(57)    Eine Einrichtung zur Entnahme von Gas- und/oder Flüssigkeitsproben aus einem Sicherheitsbehälter eines Kernkraftwerkes weist wenigstens eine im Sicherheitsbehälter (20) angeordnete, pneumatisch gesteuerte Probennahmevorrichtung (P), eine Druckluftfördereinrichtung zum Hintransport eines Probenbehältnisses zur Probennahmevorrichtung (P) und zum Rücktransport von der Probennahmevorrichtung, eine Schleuseneinrichtung (S1, S2) zum Ein- und Ausschleusen des Probenbehältnisses in den Transportkanal (T) der Druckluftfördereinrichtung und in die Probennahmevorrichtung (P) und eine Steuereinrichtung zum Steuern des Transports des Probenbehältnisses sowie der Probennahme-vorgänge auf. Diese zur ferngesteuerten Entnahme von Gas- und/oder Flüssigkeitsproben aus einem Sicherheitsbehälter eines kernkraftwerkes vorgesehene Einrichtung ist einfach im Aufbau und genügt den gebotenen Sicherheitsanforderungen.

EP 0 468 195 A1

Die Erfindung betrifft eine Einrichtung zur Entnahme von Gas- und/oder Flüssigkeitsproben aus einem Sicherheitsbehälter von Kernkraftwerken.

Für die Probennahme aus Sicherheitsbehältern von kernkraftwerken sind bisher keine Einrichtungen bekannt geworden. Es ist lediglich aus der Wiederaufarbeitungstechnik bekannt, zur Probennahme aus Verfahrenskomponenten eine evakuierte Probenflasche zu verwenden, die mit Hilfe einer Druckluftfördereinrichtung zur Probenentnahmestelle transportiert wird und dort über eine durch einen Verschluß der Flasche hindurchgestoßene Hohlnadel einer Befüllvorrichtung mit Probenmedium gefüllt wird (DE-PS 37 10 713, EP-A 0 093 609, DE-GM 77 13 201).

Die Aufgabe der vorliegenden Erfindung besteht darin, eine Einrichtung zur ferngesteuerten Entnahme von Gas- und/oder Flüssigkeitsproben aus einem Sicherheitsbehälter eines kernkraftwerkes anzugeben, die einfach im Aufbau ist und den gebotenen Sicherheitsanforderungen genügt.

Diese Aufgabe wird durch die Ausbildung gemäß Anspruch 1 gelöst.

Vorteilhafte und zweckmäßige Weiterbildungen der erfindungsgemäßen Aufgabenlösung sind in den Unteransprüchen gekennzeichnet.

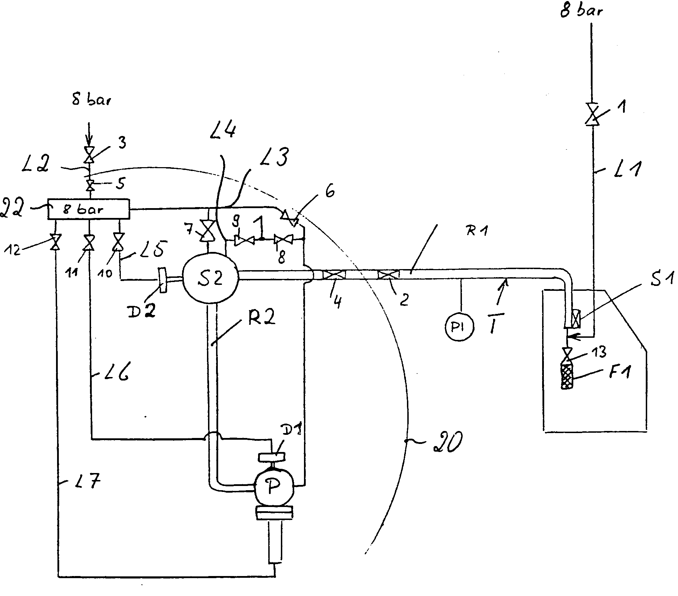

Die Erfindung soll nachfolgend anhand der beigefügten Zeichnung näher erläutert werden.

Die Zeichnung zeigt schematisch eine Einrichtung zur Entnahme von Gas- und/oder Flüssigkeitsproben aus einem Sicherheitsbehälter 20 eines Kernkraftwerkes.

Die Einrichtung umfaßt eine Druckluftfördereinrichtung mit einem Transportkanal T für ein Probenbehältnis (nicht dargestellt), der in zwei Anschnitte R1 und R2 aufgeteilt ist. Der erste Kanalanschnitt R1 führt vor. einer ersten, außerhalb des Sicherheitsbehälters gelegenen Schleuse S1 durch die Wand des Sicherheitsbehälters zu einer zweiten innerhalb des Sicherheitsbehälters befindlichen Schleuse S2, von der der zweite Kanalabschnitt R2 zu einer im Sicherheitsbehälter installierten Probenentnahmevorrichtung P führt.

Förderluft wird über eine Leitung L1 und ein Druckluft-Einsteuerventil 1 in den Transportkanal T eingeleitet zum Transport des Probenbehältnisses zur zweiten Schleuse S2.

Eingangsseitig des Transportkanals befindet sich noch ein Entlüftungsventil 13, dem ein Filter F1 nachgeschaltet ist.

Im ersten kanalabschnitt R1 befinden sich zwei Ventile 2 und 4, von denen ein Ventil (Ventil 2) außerhalb des Sicherheitsbehälters und das andere Ventil 4 innerhalb des Sicherheitsbehälters angeordnet ist.

Aus der zweiten Schleuse S2 strömt die Förderluft über ein Ventil 9 ab.

Förderluft zum Transport des Probenbehältnisses von der zweiten Schleuse S2 zur Probennahmevorrichtung P und zum Rücktransport des Probenbehältnisses von der zweiten Schleuse S2 zur eingangsseitigen Schleuse S1 wird über ein Ventil 7 der Schleuse S2 zugeführt, wobei für die Förderluft ausgangsseitig der Probennahmevorrichtung P ein Ventil 8 vorgesehen ist.

Förderluft zum Rücktransport des Probennahmebehältnisses von der Probennahmevorrichtung P zur zweiten Schleuse S2 wird über ein in einer Leitung L3 angeordnetes Ventil 6 der Probennahmevorrichtung zugeführt.

Die Ventile 8 und 9 liegen in einer die zweite Schleuse S2 und einem zwischen dem Ventil 6 und der Probennahmevorrichtung P liegenden Teil der Leitung L3 verbindende Leitung L4. Sie sind als Rückschlagventile ausgebiidet.

Die zweite Schleuse S2 weist einen Stellantrieb D2 und die Probennahmevorrichtung P einen Stellantrieb D1 für eine Probenbehältnisaufnahme auf. An beide Stellantriebe sind Druckluftleitungen L5 bzw. L6 gelegt, wobei die Stellantriebe über in diesen Druckluftleitungen angeordnete Ventile 10 bzw. 11 gesteuert mit Druckluft betätigt werden.

Die Betätigung der Probennahmevorrichtung P zur Entnahme einer Probe, d. h. zum Befüllen des Probennahmebehältnisses mit Probenmedium, erfolgt ebenfalls mit Druckluft, die über eine Leitung L7 gesteuert über ein in dieser Leitung angeordnetes Ventil 12 zugeführt wird.

Die Ventile 6, 7, 10, 11 und 12 sind an einen Druckluftverteiler 22 angeschlossen, der über eine Leitung L2 mit Druckluft versorgt wird. In dieser Leitung L2 befinden sich zwei Ventile 3 und 5, von denen eines (Ventil 3) außerhalb des Sicherheitsbehälters und das andere Ventil 5 innerhalb des Sicherheitsbehälters angeordnet ist.

An den Schleusen S1 und S2 sowie an der Probennahmevorrichtung P befinden sich Positions- und Anwesenheitsmelder für das Probenbehältnis sowie Melder zur Signalisierung des jeweiligen Zustandes der Schleusen und der Probennahmevorrichtung. Die Ausgangssignale dieser Melder werden einer Steuereinrichtung zugeführt, die die Ventile in Abhängigkeit von diesen Signalen und eines vorgegebenen Programmes steuert.

Die Funktionsweise der beschriebenen Einrichtung zur Probennahme ist wie folgt:

In der Grundstellung ist die Schleuse S1 geschlossen, befindet sich die Schleuse S2 in der Aufnahmestellung für das leere Probenbehältnis, sind sämtliche Ventile geschlossen und befindet sich die Probennahmevorrichtung P in der Probenbehältnisaufnahmestellung.

Nach dem Evakuieren des Probennahmebehältnisses wird dieses Behältnis in den ersten Kanalabschnitt R1 des Transportkanals T über die Schleuse S1 eingeführt und im kanalabschnitt R1 zur Schleuse S2 transportiert, wozu die Ventile 1, 2, 4 und 9 geöffnet werden.

Nach erfolgter Rückmeldung, daß das Probenbehältnis in der Schleuse S2 angekommen ist, wird das Probenbehältnis mit dem Antrieb D2 in den zweiten kanalabschnitt R2 eingeschleust, wozu die Ventile 3, 5 und 10 geöffnet werden.

Nach der Meldung, daß das Probenbehältnis in der Schleuse S2 in den kanalabschnitt R2 eingeschleust worden ist, werden die Ventile 2, 4, 9, 10 und 1 geschlossen und wird das Probenbehältnis im kanalabschnitt R2 zur Probennahmevorrichtung P gefördert, wobei die Ventile 7 und 8 geöffnet sind.

Nach der Meldung, daß das Probennahmebehältnis an der Probennahmevorrichtung P angekommen ist, wird das Probenbehältnis mit Hilfe des Antriebs D1 in der Probennahmevorrichtung zur Probennahme gedreht, wobei das Ventil 11 geöffnet und die Ventile 7 und 8 geschlossen werden.

Nach der Rückmeldung, daß das Probenbehältnis zur Probennahme bereit ist, erfolgt die Probennahme, wobei das Ventil 11 geschlossen und das Ventil 12 geöffnet werden. Nach Beendigung der Probennahme, wofür eine bestimmte Probennahmezeit vorgesehen sein kann, wird das Ventil 12 wieder geschlossen.

Nach der Rückmeldung, daß die Probennahme beendet ist und die Befüllhohlnadel abgezogen ist, wird das Probenbehältnis mit Hilfe des Antriebs D1 zur Abgabe aus der Probennahmevorrichtung in den kanalabschnitt R2 gedreht, wobei das Ventil 11 geöffnet wird. Das Probenbehältnis wird dann zur Schleuse S2 transportiert, nachdem die Rückmeldung erfolgt ist, daß sich das Probennahmebehältnis im kanalabschnitt R2 befindet und die Schleuse S2 empfangsbereit ist; hierzu wird das Ventil 11 geschlossen und werden die Ventile 6 und 9 geöffnet.

Sobald die Rückmeldung erfolgt ist, daß das Probenbehältnis in der Schleuse S2 angekommen ist, wird das Probenbehältnis mit Hilfe des Antriebs D2 in den ersten kanalabschnitt R1 eingeschleust, wozu das Ventil 10 geöffnet und die Ventile 6 und 9 geschlossen werden.

Nach der Rückmeldung, daß das Probenbehältnis in den Kanalabschnitt R1 eingeschleust worden ist, wird das Probenbehältnis im kanalabschnitt R1 zur eingangsseitigen Schleuse S1 gefördert, wozu das Ventil 10 geschlossen und die Ventile 7, 4, 2 und 13 geöffnet werden. Wenn die Rückmeldung erfolgt, daß sich das Probennahmebehältnis in der Schleuse S1 befindet, werden die Ventile 7, 4 und 2 geschlossen.

Nachdem der Förderdruck über das Ventil 13 und das Filter F1 abgebaut ist, wird die oben erläuterte Grundstellung angefahren, in der das Probennahmebehältnis über die Schleuse S1 entnommen werden kann zwecks Zuleitung zu einer Untersuchungsstation.


**Patentansprüche**

1.  Einrichtung zur Entnahme von Gas- und/oder Flüssigkeitsproben aus einem Sicherheitsbehälter eines kernkraftwerkes,

    **gekennzeichnet durch**

    wenigstens eine im Sicherheitsbehälter (20) angeordnete, pneumatisch gesteuerte Probennahmevorrichtung (P),

    eine Druckluftfördereinrichtung zum Hintransport eines Probenbehältnisses zur Probennahmevorrichtung (P) und zum Rücktransport von der Probennahmevorrichtung,

    eine Schleuseneinrichtung (S1, S2) zum Ein- und Ausschleusen des Probenbehältnisses in den Transportkanal (T) der Druckluftfördereinrichtung und in die Probennahmevorrichtung (P),

    eine Steuereinrichtung zum Steuern des Transports des Probenbehältnisses sowie der Probennahmevorgänge.

2.  Einrichtung nach Anspruch 1, **dadurch gekennzeichnet**, daß die Druckluftfördereinrichtung einen in zwei Abschnitte (R1 und R2) aufgeteilten Transportkanal (T) aufweist, daß eine erste Schleuse (S1) eingangseitig des Transportkanals zum Ein- und Ausschleusen eines Probenbehältnisses und eine zweite Schleuse (S2) an der übergangsstelle vom ersten kanalabschnitt (R1) zum zweiten zur Probennahmevorrichtung (P) führenden kanalabschnitt (R2) zum Ein- und Ausschleusen des Probenbehältnisses in den zweiten Kanalabschnitt (R2) und zum Transport des Probenbehältnisses zur Probennahmevorrichtung (P) und zurück vorgesehen sind.

3.  Einrichtung nach Anspruch 1 oder 2, **dadurch gekennzeichnet**, daß wenigstens die zweite Schleuse (S2) einen pneumatischen Stellantrieb (D2) aufweist, der über ein Ventil (10) gesteuert wird.

4. Einrichtung nach Anspruch 1 oder 2, **dadurch gekennzeichnet**, daß die Probennahmevorrichtung (P) einen pneumatisch über ein Ventil (11) gesteuerten Stellantrieb (D1) für die Einstellung des Probenbehältnisses in die Aufnahme-, Befüll- und Abgabestellung aufweist, und daß ferner die Probennahmevorrichtung (P) zur Probennahme über ein Ventil (12) gesteuert mit Druckluft beaufschlagbar ist.

5. Einrichtung nach einem der vorhergehenden Ansprüche, **dadurch gekennzeichnet**, daß zur Steuerung des Transportes des Probenbehältnisses durch den pneumatischen Transportkanal im ersten kanalabschnitt (R1) zur zweiten Schleuse (S2) ein in einer Förderluftleitung (L1) angeordnetes Förderluft-Einsteuerventil (1) und ein Ventil (9) ausgangsseitig der zweiten Schleuse (S2) vorgesehen sind,

und daß zwei weitere Ventile (2 und 4) vorgesehen sind, von denen eines außerhalb des Sicherheitsbehälters (20) und das andere innerhalb des Sicherheitsbehälters angeordnet ist,

und daß zur Steuerung des Transportes des Probenbehältnisses von der zweiten Schleuse (S2) zur ersten Schleuse (S1) eingangsseitig der zweiten Schleuse (S2) ein Ventil (7), ferner die beiden Ventile (2 und 4) und ausgangsseitig der ersten Schleuse (S1) ein Entlüftungsventil (13) mit nachgeschaltetem Filter (F1) vorgesehen sind.

6. Einrichtung nach einem der vorhergehenden Ansprüche, **dadurch gekennzeichnet**, daß zur Steuerung des Transportes des Probenbehältnisses durch den zweiten Kanalabschnitt (R2) von der zweiten Schleuse (S2) zur Probennahmevorrichtung (P) das schleuseneingangsseitige Ventil (7) und ausgangsseitig der Probennahmevorrichtung (P) ein Ventil (8)

und zur Steuerung des Transports des Probenbehältnisses von der Probennahmevorrichtung (P) zurück zur Schleuse (S2) ein Förderlufteinsteuerventil (6) und das schleusenausgangsseitige Ventil (9) vorgesehen sind.

7. Einrichtung nach einem der vorhergehenden Ansprüche, **dadurch gekennzeichnet**, daß die Ventile (12, 11, 10, 7, 6) zur Versorgung der zweiten Schleuse (S2) und der Probennahmevorrichtung (P) mit Druckluft an einen Druckluftverteiler (22) angeschlossen sind, in den eine Druckluftzuführleitung (L2) führt, in die zwei steuerbare Ventile (3, 5) geschaltet sind, von denen eines außerhalb und das andere innerhalb des Sicherheitsbehälters (20) angeordnet ist.

8. Einrichtung nach einem der vorhergehenden Ansprüche, **dadurch gekennzeichnet**, daß in den Schleusen (S1, S2) und in der Probennahmevorrichtung (P) Melder zur Signalisierung des Vorhandenseins und-/oder der jeweiligen Position des Probenbehältnisses sowie des jeweiligen Zustandes der Schleusen und der Probennahmevorrichtung angeordnet sind, deren Ausgangssignale der Steuereinrichtung zugeführt sind, die die Schleusen, die Ventile für den Probenbehältnistransport und die Betätigung der Probennahmevorrichtung in Abhängigkeit von diesen Meldersignalen nach einem vorgegebenen Programm steuert.

9. Einrichtung nach einem der vorhergehenden Ansprüche, **dadurch gekennzeichnet**, daß eine Einrichtung zum Evakuieren des Probenbehältnisses vor dem Einschleusen desselben in den Transportkanal (T) vorgesehen ist.

EP 0 468 195 A1

| Europäisches Patentamt | EUROPÄISCHER RECHERCHENBERICHT | Nummer der Anmeldung |
|---|---|---|
| | | EP 91 10 9877 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 093 609 (BRITISH NUCLEAR FUELS LTD)<br>* Seite 3, Zeilen 9-14,22-24; Seite 16, Zeile 20 - Seite 18, Zeile 9; Figuren 8-9 * | 1-4,8-9 | G 21 C 17/022<br>G 21 F 7/06<br>G 01 N 1/10 |
| A | US-A-4 160 382 (FINSTERWALDER et al.)<br>* Zusammenfassung; Spalte 4, Zeilen 18-28; Figur 1 * | 1-2,4 | |
| A | US-A-4 662 231 (SCHAARSCHMIDT et al.)<br>* Spalte 4, Zeilen 7-27; Spalte 5, Zeilen 13-20; Figur * | 1-2,4 | |
| A | DE-A-3 142 031 (GENERAL ELECTRIC)<br>* Zusammenfassung; Figur 1 * | 1-2,4-6 | |
| | | | **RECHERCHIERTE SACHGEBIETE (Int. Cl.5)**<br><br>G 21 C<br>G 01 N<br>B 01 L<br>G 21 F |

Der vorliegende Recherchenbericht wurde für alle Patentansprüche erstellt

| Recherchenort | Abschlußdatum der Recherche | Prüfer |
|---|---|---|
| DEN HAAG | 25-10-1991 | DEROUBAIX P.G.M. |